# EUROPÄISCHE PATENTSCHRIFT

(11) **EP 1 230 894 B1**
(45) Veröffentlichungstag und Bekanntmachung des Hinweises auf die Patenterteilung: **12.09.2007**
(21) Anmeldenummer: 02002034.3
(22) Anmeldetag: 07.02.2002
(51) Int. Cl.: G06F 19/00, A61B 5/16, A61B 5/0484

(54) **Vorrichtung, Verfahren und Computerprogrammprodukt zum Messen einer körperlichen oder physiologischen Aktivität einer Testperson und zum Beurteilen des psychosomatischen Zustands der Testperson**
Device,method and computerprogram for measuring a physical and physiological activity of a test person and for evaluating the psychosomatic state of the test person
Dispositif, méthode et programme d'ordinateur pour mesurer l'activité physique et physiologique d'un patient et pour l'évaluation de l'état psychosomatique de cette personne

(30) Priorität: 09.02.2001 DE 10105965
(43) Veröffentlichungstag der Anmeldung: 14.08.2002
(73) Patentinhaber: m-tec AG, 82054 Altkirchen bei München (DE)
(72) Erfinder: von Buengner, Peter-Raphael c/o m-tec AG, 82054 Altkirchen bei München (DE)
(74) Vertreter: Schwabe - Sandmair - Marx

(56) Entgegenhaltungen:
- WO-A-91/12578
- DE-A- 2 730 254
- DE-A- 4 031 084
- DE-A- 4 110 049
- DE-A- 4 139 241
- US-A- 3 691 652
- US-A- 3 893 450
- US-A- 5 467 777
- US-A- 5 511 982

## Beschreibung

Die vorliegende Erfindung betrifft eine Vorrichtung und ein Verfahren sowie ein Computerprogrammprodukt zum Erfassen von Daten betreffend dem psychosomatischen Zustand einer Testperson, insbesondere zum Messen einer körperlichen oder physiologischen Aktivität einer Testperson und zum Beurteilen des psychosomatischen Zustands der Testperson.

Die menschliche Muskulatur umfasst quergestreifte und längsgestreifte Muskeln, die einen Muskeltonus aufweisen. Wird eine Testperson einem Sinnesreiz ausgesetzt, wird ihr beispielsweise ein mit positiven oder negativen Assoziationen verknüpftes Bild oder Geräusch dargeboten, so wird der Muskeltonus schwächer oder stärker, je nach dem, ob die Testperson positiv oder negativ auf den dargebotenen Sinnesreiz reagiert. Die Änderung des Muskeltonus gibt somit Aufschluss über den psychosomatischen Zustand einer Testperson.

In der medizinischen Diagnostik ist das Verfahren der sogenannten Kinesiologie bekannt, bei dem mit einer Testperson ein manueller Muskeltest durchgeführt wird. In der Regel soll die Testperson versuchen, ihren Arm hochzuhalten, während eine weitere Person, beispielsweise ein Arzt oder Therapeut, versucht, den ausgestreckt gehaltenen Arm herunterzudrücken. Je nach dem, wie leicht oder schwer sich der Arm der Testperson nach unten drücken lässt, erhält man Aufschluss darüber, ob der Patient auf das, womit er gerade konfrontiert wurde, positiv reagiert oder nicht.

Die auftretenden Kräfte müssen von dem Arzt oder Therapeuten subjektiv abgeschätzt werden. Außerdem sind die Tests in der Kinesiologie vergleichsweise mühsam und zeitaufwendig, da der Arzt oder Therapeut sich die subjektiv geschätzten Reaktionen entweder merken muss oder bei Darbietung einer Anzahl verschiedener Sinnesreize sich die jeweiligen Reaktionen zwischenzeitlich notieren muss. Es besteht somit ein Bedürfnis nach objektiveren und einfacher und schneller auszuführenden Testverfahren und -vorrichtungen.

In der Regel sind die vorgenannten Tests auch vergleichsweise teuer, da eine gesicherte Diagnose einen erfahrenen Therapeuten oder Arzt erfordert.

Aus dem Stand der Technik sind Biofeedback-Geräte bekannt, die von einer körperlichen oder physiologischen Aktivität einer Testperson ein elektrisches Signal abgreifen, das beispielsweise den aktuellen Puls oder Blutdruck darstellt. Das Signal wird elektronisch weiter verarbeitet und der Testperson dargeboten, die nun durch Feedback versuchen soll, das elektrische Signal und somit die zugrunde liegende körperliche oder physiologische Aktivität zu beeinflussen. Solche Geräte können dazu verwendet werden, um das Stressverhalten von Testpersonen durch Feedback zu verbessern.

Aufgabe der vorliegenden Erfindung ist es, eine Vorrichtung und ein Verfahren zu schaffen, so dass in einfacher und zuverlässiger Weise für eine Testperson Daten erfasst werden können, die als Grundlage zur Beurteilung des psychosomatischen Zustands der Testperson dienen können. Gemäß der Erfindung soll auch ein Computerprogrammprodukt zum Durchführen des erfindungsgemäßen Verfahrens auf einen Computer geschaffen werden. Die Erfindung wird durch den Gegenstand der Ansprüche 1, 10 und 12 definiert.

Gemäß der Erfindung wird eine Vorrichtung zum Erfassen von Daten betreffend den psychosomatischen Zustand einer Testperson geschaffen, insbesondere zum Messen einer körperlichen oder physiologischen Aktivität einer Testperson und zum Beurteilen des psychosomatischen Zustands der Testperson, welche Vorrichtung einen Reizgenerator, um der Testperson Sinnesreize darzubieten, eine Steuereinrichtung, die den Reizgenerator so steuert, dass der Testperson automatisch eine Sequenz unterschiedlicher Sinnesreize dargeboten wird, einen Sensor, um von einer körperlichen oder physiologischen Aktivität der Testperson in Reaktion auf einen dargebotenen Sinnesreiz ein elektrisches Signal abzuleiten, und einen Komparator umfasst, um das elektrische Signal mit einem vorgebbaren Sollwert zu vergleichen.

Vorteilhaft ist, dass in automatisierter Weise Daten von einer Testperson gewonnen werden können, die Aufschluss über den psychosomatischen Zustand der Testperson geben können. Um solche Daten zu gewinnen, bedarf es gemäß der Erfindung somit keines Arztes oder Therapeuten. Vielmehr kann die Vorrichtung selbsttätig an der Testperson Tests durchführen. Die dabei gewonnenen Daten können als Grundlage für eine nachfolgende Beurteilung des psychosomatischen Zustands der Testperson dienen. Hierfür bedarf es jedoch nicht zwingend eines Arztes oder Therapeuten. Vorteilhaft ist auch, dass die Daten schneller gewonnen werden können, da die Sinnesreize automatisiert dargeboten werden. Vorteilhaft ist ferner, dass die Daten objektiver sind, da sie nicht mehr durch das subjektive Empfinden eines Arztes oder Therapeuten verfälscht werden können. Somit lassen die Daten gemäß der Erfindung eine gesichertere Diagnose zu.

Als Sinnesreize können sämtliche Reize verwendet werden, die dazu geeignet sind, um bei einer Testperson eine körperliche oder physiologische Aktivität hervorzurufen. Bevorzugt werden gemäß der Erfindung visuelle Reize, beispielsweise Bilder, Farben oder Texte, akustische Reize, beispielsweise Töne, Melodien, Geräusche oder gesprochene Wortsequenzen, taktile Reize, beispielsweise durch Druckaktuatoren hervorgerufene Berührungsreize, verwendet. Grundsätzlich können jedoch auch Reize verwendet werden, die auf Geschmacksnerven oder Riechnerven der Testperson einwirken, wozu die Reize entweder in natürlicher Form, beispielsweise als Geruch, hervorgerufen werden oder mittels an geeigneter Stelle angebrachter Elektroden.

Besonders bevorzugt wird als Steuereinrichtung ein Computer bzw. ein Datenverarbeitungsmittel verwendet, insbesondere ein tragbarer Computer, der gleichzeitig auch als Reizgenerator zum Erzeugen der Sinnesreize dienen kann. Somit wird gemäß der Erfindung eine besonders einfache und kostengünstige Vorrichtung geschaffen.

Das von dem Sensor abgeleitete elektrische Signal wird mittels eines Komparators mit einem Sollwert verglichen. Dieser Sollwert kann gemäß der Erfindung vorgegeben werden und wird besonders bevorzugt an das Aktivitätsmuster der Testperson individuell angepasst. Während die Sequenz von Sinnesreizen dargeboten wird, hat die Testperson die Aufgabe, auf einen Sinnesreiz jeweils so zu reagieren, dass die Stärke der körperlichen oder physiologischen Aktivität dem Schwellenwert entspricht. Die Stärke der Reaktion wird der Testperson dabei nicht angezeigt. Aus der Differenz zwischen der Stärke der detektierten körperlichen oder physiologischen Aktivität der Testperson in Reaktion auf einen dargebotenen Sinnesreiz und dem vorgegebenen Schwellenwert kann ein Datensatz abgeleitet werden, der anzeigt, ob die Aktivität in Reaktion auf den dargebotenen Sinnesreiz vergleichsweise stark oder schwach ausgefallen ist. Diese Information kann in einem nachfolgenden Beurteilungsschritt dazu verwendet werden, um den psychosomatischen Zustand der Testperson zu beurteilen. Insbesondere kann beurteilt werden. ob der Sinnesreiz bei der Testperson eine positive oder negative Reaktion hervorgerufen hat.

Da der Testperson nicht ein einziger Sinnesreiz, sondern vielmehr eine Sequenz unterschiedlicher Sinnesreize dargeboten wird, können etwaige Schwankungen in der körperlichen oder physiologischen Aktivität der Testperson herausgefiltert oder herausgemittelt werden. Insbesondere können in der Sequenz Wiederholungen ein und desselben Sinnesreizes enthalten sein, so dass für eine körperliche oder physiologische Aktivität der Testperson in Reaktion auf ein und denselben Sinnesreiz über mehrere Messwerte gemittelt werden kann, um einen Mittelwert für die jeweilige Aktivität zu erhalten. Vorteilhaft ist, dass somit die abgeleiteten Daten eine noch bessere Grundlage zur Beurteilung des psychosomatischen Zustands darstellen.

Die Steuereinrichtung umfasst eine Datenbank, die Datensätze enthält, die den darzubietenden Sinnesreizen entsprechen, die der Testperson dargeboten werden sollen. Die Datensätze können in einem Steuermenü von einer Bedienperson ausgewählt werden, so dass eine individuelle Sequenz für die jeweilige Testperson erstellt werden kann. Die Datenbank kann auch in elektronischer Form vorliegen und auf einem Datenspeicher, beispielsweise einer CD-ROM, abgespeichert sein.

Zweckmäßig kann auch eine Eingabevorrichtung vorgesehen sein, um in die Datenbank weitere Datensätze einzugeben, die für Sinnesreize stehen, die der Testperson dargeboten werden sollen. Sollen der Testperson beispielsweise Texte dargeboten werden, so können weitere Texte über eine Tastatur der Steuereinrichtung eingegeben werden. Als Eingabevorrichtung kann jedoch auch eine Eingabe-/Ausgabeschnittstelle zu externen Reizgeneratoren dienen, beispielsweise zu Ton- oder Tonsequenzgeneratoren, Sprachaufzeichnungsgeräten, Grafikeingabegeräten und dergleichen.

Die Sequenz von Sinnesreizen kann mit fest vorgegebener oder mit variabel vorgebbarer Geschwindigkeit, insbesondere angepasst an die Reaktionsfähigkeit der Testperson, ablaufen. Besonders bevorzugt steuert die Steuereinrichtung den Reizgenerator jedoch so, dass ein nächster Reiz aus der Sequenz von Sinnesreizen erst dann dargeboten wird, wenn die Testperson auf einen vorherigen Reiz der Sequenz reagiert hat, was besonders bevorzugt dadurch festgestellt wird, dass die Stärke eines in Reaktion auf den vorhergehenden Reiz erfassten elektrischen Signals, das der körperlichen oder physiologischen Aktivität der Testperson in Reaktion auf den zuvor dargebotenen Sinnesreiz entspricht, einen vorgegebenen Schwellenwert überschreitet. Vorteilhaft ist, dass durch den Schwellenwert vermieden werden kann, dass versehentlich ein Rauschen oder eine Aktivität, die nicht in Reaktion auf einen Sinnesreiz festgestellt wird, als elektrisches Signal erfasst wird, das weiter verarbeitet wird.

Grundsätzlich eignen sich als Sensoren sämtliche Arten von Sensoren, mit denen körperliche oder physiologische Aktivitäten bei der Testperson überwacht werden können. Besonders bevorzugt werden gemäß der Erfindung Drucksensoren verwendet, zur Aufnahme von Druckkräften, wenn die Testperson auf den Drucksensor eine Kraft ausübt. Diese Art der Erfassung von Aktivitäten der Testperson ist somit an die aus der Kinesiologie bekannte Vorgehensweise angelehnt. Besonders bevorzugt wird als Drucksensor eine Waage verwendet, insbesondere eine Briefwaage, die auch einen A/D-Wandler und eine Schnittstelle zur Weiterleitung der Detektorsignale an eine Auswerteeinheit umfassen kann. Als Sensoren können gemäß der Erfindung alternativ oder ergänzend auch EEG-Sensoren, EMG-Sensoren oder dergleichen verwendet werden. Aus den so gewonnenen Aktivitätsmustern, beispielsweise Nervenimpulsmustern, lassen sich ebenfalls Rückschlüsse auf den psychosomatischen Zustand der Testperson gewinnen.

Zur Aufbereitung und Weiterverarbeitung der gewonnenen Daten ist bevorzugt eine Prozessoreinheit, insbesondere ein Mikroprozessor, vorgesehen, so dass die gewonnenen Daten grafisch aufbereitet und auf einer Anzeige angezeigt werden können und/oder als Datensatz abgespeichert werden können, insbesondere auf einem austauschbaren Datenträger. Die gewonnenen Daten können somit noch einfacher analysiert werden.

Zum Vorgeben des Sollwerts, den die körperliche oder physiologische Aktivität der Testperson in Reaktion auf einen dargebotenen Sinnesreiz jeweils erreichen soll, ist bevorzugt eine Sollwerteinheit vorgesehen, die eine visuelle Anzeige zum Anzeigen der Stärke der körperlichen oder physiologischen Aktivität der Testperson in Reaktion auf einen Test-Sinnesreiz und eine visuelle Anzeige zum Vorgeben eines Sollwerts aufweist. Der Testperson wird zumindest ein Test-Sinnesreiz dargeboten, mit der Aufgabe so zu reagieren, dass die Stärke der gemessenen Aktivität in Reaktion auf den zumindest einen Sinnesreiz den visuell angezeigten Sollwert erreicht.

Wenn die Sequenz unterschiedlicher Sinnesreize der Testperson dargeboten wird, hat die Testperson die Aufgabe, auf jeden Sinnesreiz möglichst gleich und in der Stärke des vorgegebenen Sollwerts zu reagieren. Somit kann in besonders einfacher Weise jegliche Abweichung der tatsächlichen Stärke der Aktivität von dem Sollwert ermittelt werden.

Da es vorkommen kann, dass die Testperson während der Sequenz ermüdet, steuert die Steuereinrichtung bevorzugt die Sollwerteinheit jedes Mal dann an, wenn die Abweichung der Stärke der körperlichen oder physiologischen Aktivität von dem Sollwert einen vorgegebenen Schwellenwert eine Anzahl von Malen überschreitet. Reagiert die Testperson zufällig vergleichsweise stark oder schwach, so löst dies noch nicht die Festlegung eines neuen Sollwerts aus. Vorteilhaft ist, dass das Verfahren und die Vorrichtung gemäß der Erfindung in einfacher Weise einer Ermüdung oder einem erhöhten Aktivitätspegel der Testperson Rechnung tragen kann.

Es ist bekannt, dass gewisse Testpersonen auch genau entgegengesetzt reagieren können. Man sagt von solchen Testpersonen, dass sie im Reverse seien. Würde eine normale Testperson beispielsweise im Falle einer positiven Reaktion auf einen Sinnesreiz besonders heftig reagieren, so reagieren Testpersonen im Reverse genau entgegengesetzt, d.h. relativ schwach. Dies gilt auch für den umgekehrten Fall einer negativen Reaktion. Um solche Testpersonen, die im Reverse sind, herauszufiltern, ist die Steuereinrichtung bevorzugt so ausgelegt, dass der Testperson auch eine objektiv falsche Information, d.h. eine Information, von der man gesichert weiß, dass diese nicht zutrifft, dargeboten werden kann, wobei die Stärke der körperlichen oder physiologischen Aktivität in Reaktion auf die falsche Information dann mit dem zuvor festgelegten Schwellenwert verglichen wird. Wird festgestellt, dass es sich bei der Testperson um eine Testperson im Reverse handelt, so werden gemäß der Erfindung die gewonnenen Daten genau umgekehrt ausgewertet, d.h. Daten für vergleichsweise schwache Reaktionen werden in Daten für vergleichsweise starke Reaktionen umgewandelt und umgekehrt.

Zur Erzeugung von objektiv falschen Informationen ist die Steuereinrichtung bevorzugt mit einer Datenbank mit für die Testpersonen spezifischen Datensätzen verknüpft, beispielsweise einer CD-ROM, die objektiv falsche und/oder objektiv richtige Informationen enthält und aus denen die Steuereinrichtung dann die der Testperson darzubietende subjektiv falsche Information generiert.

Gemäß einem weiteren Gesichtspunkt der Erfindung wird ein Verfahren zum Erfassen von Daten betreffend dem psychosomatischen Zustand einer Testperson geschaffen, insbesondere zum Messen einer körperlichen oder physiologischen Aktivität einer Testperson und zum Beurteilen des psychosomatischen Zustands der Testperson, bei welchem Verfahren der Testperson Sinnesreize dargeboten werden und elektrische Signale von einer körperlichen oder physiologischen Aktivität der Testperson in Reaktion auf einen dargebotenen Sinnesreiz abgeleitet werden und die elektrischen Signale mit einem vorgegebenen Sollwert verglichen werden, wobei die Sinnesreize der Testperson in Form einer Sequenz unterschiedlicher Sinnesreize automatisch dargeboten werden.

Das Verfahren gemäß der Erfindung ist kein diagnostisches oder therapeutisches Verfahren. Vielmehr dient das Verfahren gemäß der Erfindung zum Ableiten von Daten, die als Grundlage für eine nachfolgende Auswertung bzw. Beurteilung dienen, die nicht von Ärzten oder Therapeuten vorgenommen werden muss.

Gemäß einem weiteren Gesichtspunkt der Erfindung wird auch ein Computerprogrammprodukt geschaffen, das Programmkode- bzw. Softwarekodeabschnitte aufweist, die direkt in den Arbeitsspeicher eines Computers bzw. Datenverarbeitungsmittel ladbar sind, der bzw. das mit zumindest einem Sensor zum Erfassen von elektrischen Signalen von einer körperlichen oder physiologischen Aktivität einer Testperson in Reaktion auf einen Sinnesreiz verbunden ist, so dass der Computer das erfindungsgemäße Verfahren ausführt.

Eine besonders bevorzugte Verwendung der erfindungsgemäßen Vorrichtung, des erfindungsgemäßen Verfahrens und Computerprogrammprodukts betrifft die Verwendung zum Testen von Allergien. Dabei werden der Testperson Bilder und/oder Texte dargeboten, die zu testende, bei der Testperson potentiell eine Allergie auslösende Substanzen repräsentieren. Aus der körperlichen oder physiologischen Aktivität in Reaktion auf die Sequenz der dargebotenen Reize kann auf besondere Allergien, aber auch Vorlieben der Testperson geschlossen werden. Reagiert eine Testperson beispielsweise besonderes markant auf Bilder oder auf die Worte von gewissen Produkten, so kann aus den gewonnenen Daten auf eine Allergie auf diese Produkte rückgeschlossen werden. Vorteilhaft ist, dass ein solcher Allergietest besonders preisgünstig ist und rasch ausgeführt werden kann.

Nachfolgend wird die Erfindung in beispielhafter Weise und unter Bezugnahme auf die beigefügten Figuren beschrieben. Es zeigen:
- Figur 1: eine Vorrichtung gemäß der Erfindung mit einer Anzahl beispielhafter Sensoren zum Erfassen von Signalen aus Reaktionen oder Aktivitäten im Handbereich einer Testperson;
- Figur 2: in schematischer Weise ein Flussdiagramm eines Verfahrens gemäß der Erfindung;
- Figur 3: Verfahrensschritte, die vor den Verfahrensschritten gemäß Figur 2 ausgeführt werden können;
- Figur 4: zwei Beispiele für mögliche Bildschirmanzeigen, die die Reaktion einer Testperson auf den Reiz "Milch" wiedergeben; und
- Figur 5: ein weiteres Beispiel für eine bevorzugte Verwendungsform der erfindungsgemäßen Vorrichtung.

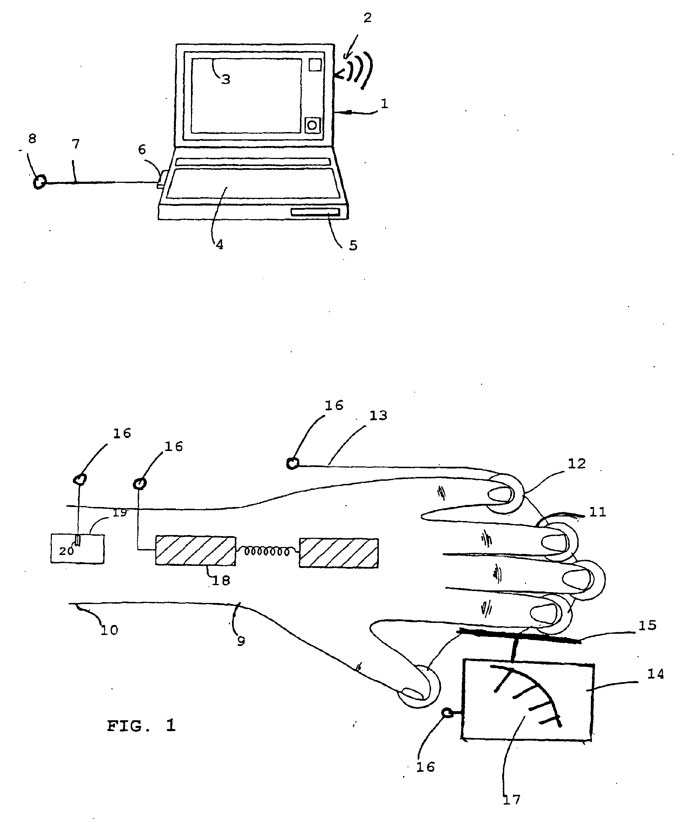

Figur 1 zeigt eine Vorrichtung gemäß der Erfindung. Diese umfasst einen Computer 1 und zumindest einen Sensor, um von einer körperlichen oder physiologischen Aktivität der Testperson im Bereich der Hand 9 bzw. des Unterarms 10 in Reaktion auf einen dargebotenen Sinnesreiz ein elektrisches Signal abzuleiten. Der Computer 1 dient zugleich als Reizgenerator und als Steuereinrichtung. Grundsätzlich können Reizgenerator und Steuereinrichtung auch getrennte Einheiten bilden. Der Computer 1 hat einen Bildschirm 3 zur Anzeige visueller Reize, beispielsweise von Grafiken, Bildern oder Texten, und einen Lautsprecher 2 zur Ausgabe akustischer Reize, beispielsweise von Tönen, Geräuschen, Melodien oder gesprochenen Texten.

Auf dem Computer ist eine Datenbank installiert, mit Datensätzen, die die der Testperson darzubietenden Sinnesreize repräsentieren, beispielsweise Datensätzen für Bilder, Grafiken, Farben, Töne, Melodien, Geräusche, Texte und dergleichen. Die Datenbank kann vorinstalliert sein und auch mehrere Arten von Sequenzen von Sinnesreizen enthalten, die der Benutzer über die Tastatur 4 und ein Menü auswählen kann. Der Benutzer kann die Sequenz darzubietender Sinnesreize auch individuell zusammenstellen und als Datenbank oder Datei abspeichern. Hierzu wählt der Benutzer über die Tastatur 4 oder eine Maus (nicht gezeigt) aus einem Menü auf dem Bildschirm 3 die darzubietenden Sinnesreize individuell aus. Zum Abspeichern dient das schematisch dargestellte Laufwerk 5, beispielsweise für eine Floppy-Disk, eine Wechselfestplatte oder eine schreib-/lesbare Disk.

Nach Betätigen eines Startsignals spielt der Computer 1 die Sequenz von Sinnesreizen aus der Datenbank automatisch ab. Dabei kann die Sequenz mit fest vorgegebener Geschwindigkeit oder mit variabler Geschwindigkeit ablaufen, wobei die Geschwindigkeit insbesondere an das Reaktionsvermögen der jeweiligen Testperson angepasst werden kann. Es kann auch vorgesehen sein, dass ein nächster Reiz aus der Sequenz erst dann dargeboten wird, wenn die Reaktion der Testperson auf den vorhergehenden Reiz einen vorgegebenen Schwellenwert überschritten hat. Hierzu enthält die Software des Computers 1 eine Diskriminator-Routine zum Auswerten der erfassten elektrischen Signale.

Der Computer weist ferner eine Schnittstelle 6 auf, über die analoge oder digitale Messdaten von den Sensoren in den Computer 1 eingegeben werden können. Die Kabelanschlüsse 16 der Sensoren stehen mit dem Kabelende 8 des Verbindungskabels 7 in Verbindung, das mit der Schnittstelle 6 des Computers 1 verbunden ist.

Der untere Teil von Figur 1 zeigt eine menschliche Hand 9 mit Fingern 11 und einem Unterarm 10. Dargestellt sind eine beispielhafte Auswahl verschiedener Sensoren, die zum Ableiten elektrischer Signale von einer körperlichen oder physiologischen Aktivität der Testperson dienen können. Hierzu sind unter den Fingerkuppen Drucksensoren 12 angeordnet. Beim Darbieten der Sequenz kann der Testperson die Aufgabe gestellt werden, mit einer möglichst konstanten Kraft auf die Drucksensoren 12 zu drücken. Die Drucksensoren 12 sind über das Kabel 13 miteinander verbunden, können jedoch auch einzeln über jeweilige Kabelanschlüsse 16 mit dem Kabelanschluss 8 des Kabels 7 in Verbindung stehen, zur drahtgebundenen oder drahtlosen Übertragung der elektrischen Signale an die Schnittstelle 6 des Computers 1.

Unterhalb der Hand 9 ist schematisch eine Briefwaage 14 dargestellt, mit einer Waagenfläche 15 (die als Druckaufnehmer dient) und einer Skala 17, die während des Ablaufens der Sequenz von Sinnesreizen der Testperson nicht sichtbar sein sollte. Die Waage 14 übermittelt die Daten drahtgebunden oder drahtlos an den Computer 1. Hierzu kann eine Schnittstelle 16, insbesondere eine RS 232- oder USB-Schnittstelle, vorgesehen sein, die mit der Schnittstelle 6 des Computers 1 in Verbindung steht. Während des Ablaufens der Sequenz von Sinnesreizen hat die Testperson die Aufgabe, in Reaktion auf einen Sinnesreiz jeweils mit einer möglichst konstanten Kraft auf die Waage 14 zu drücken, entsprechend dem in den Verfahrensschritten S8 oder S10 vorgegebenen Schwellenwert.

Statt der Drucksensoren 12, 14, 15 oder in Ergänzung zu diesen können weitere Sensoren im Bereich der Hand 9, des Unterarms 10 oder anderer Körperpartien, beispielsweise des Oberschenkels der Testperson, vorgesehen sein. Lediglich beispielhaft ist in Figur 1 zusätzlich ein Goniometer 18 im Bereich der Handgelenks dargestellt, das die Winkelabweichung des Handgelenks von einer neutralen Position in zwei zueinander senkrechten Richtungen misst und diese über die Schnittstelle 16 und die Schnittstelle 6 an den Computer 1 übermittelt. Zusätzlich kann ein EMG-Sensor 19, 20 auf die Hautoberfläche aufgebracht sein, der die von einem Muskel hervorgerufene elektrische Aktivität misst. Außerdem können EEG-Sensoren (nicht gezeigt) an diversen Körperpartien angeordnet sein, die Nervenimpulsmuster abgreifen, um diese an den Computer 1 zu übermitteln.

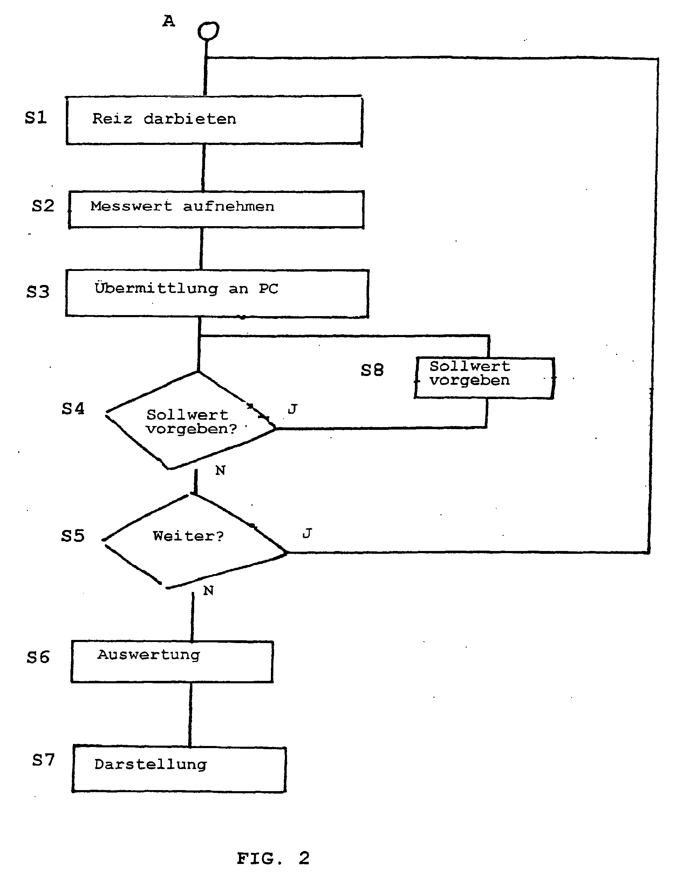

Auf dem Computer 1 ist eine Software installiert, die die nachfolgend anhand der Figuren 2 bis 4 beschriebenen Verfahrensschritte ausführt.

Figur 2 zeigt ein beispielhaftes Flussdiagramm zum Ausführen eines Verfahrens gemäß der Erfindung. In der Schleife, umfassend die Verfahrensschritte S1 bis S5, wird der Testperson eine Sequenz unterschiedlicher Sinnesreize dargeboten. Die Schleife umfasst den Verfahrensschritt S1, wo mit Hilfe eines Reizgenerators, insbesondere in Form eines Computers, ein Sinnesreiz dargeboten wird. Im Verfahrensschritt S2 wird mittels der beispielhaft in Figur 1 angedeuteten Sensoren ein elektrisches Signal von einer körperlichen oder physiologischen Aktivität der Testperson in Reaktion auf den im Verfahrensschritt S1 dargebotenen Sinnesreiz abgeleitet. Das elektrische Signal wird anschließend im Verfahrensschritt S3 in analoger oder digitaler Form an den Computer 1 übermittelt. Dort erfolgt anschließend die Auswertung der elektrischen Signale im Verfahrensschritt S6 und die Aufbereitung und Darstellung der gewonnenen Daten im Verfahrensschritt S7.

Die Schleife aus den Verfahrensschritten S1 bis S5 läuft bis zum Ende der Sequenz ab. Hierzu wird im Verfahrensschritt S5 abgefragt, ob das Ende der Sequenz bereits erreicht ist oder nicht. Falls das Ende noch nicht erreicht ist, kehrt das Verfahren zurück zum Verfahrensschritt S1.

Während die Sequenz von Sinnesreizen abläuft, kann die Auswertung bereits vorgenommen werden. Insbesondere kann im Verfahrensschritt S4 festgestellt werden, in welchem Ausmaß die Stärke des abgeleiteten elektrischen Signals von dem vorgegebenen Schwellenwert abweicht. Wenn die Abweichung der Stärke des elektrischen Signals von dem vorgegebenen Sollwert einen Schwellenwert überschreitet, geht das Verfahren über zum Verfahrensschritt S8, wo ein neuer Sollwert vorgegeben wird bzw. der Testperson der bisherige Sollwert erneut antrainiert wird, was nachfolgend anhand des Verfahrensschrittes S10 noch beschrieben werden wird. Im Anschluss an den Verfahrensschritt S8 kehrt das Verfahren dann zurück zur Schleife S1 bis S5.

Somit wird der Testperson eine Sequenz unterschiedlicher Sinnesreize dargeboten. In der Sequenz können auch Wiederholungen in regelmäßigen oder unregelmäßigen, insbesondere zufallsbestimmten, Abständen auftreten. Die Testperson hat nun die Aufgabe, jeweils mit einer zuvor vorgegebenen Stärke auf einen Sinnesreiz zu reagieren. Beispielsweise kann die Testperson die Aufgabe erhalten, in Reaktion auf einen Sinnesreiz jeweils mit einer Druckkraft von beispielsweise etwa 500 g auf die in Figur 1 gezeigte Briefwaage 14 oder die Fingerdrucksensoren 12 zu drücken. Die Testperson erhält zuvor die Gelegenheit, zu erfühlen, wie stark die Soll-Druckkraft von 500 g ist, was nachfolgend noch anhand des Verfahrensschrittes S10 beschrieben werden wird.

Während die Sequenz der Sinnesreize abläuft, hat die Testperson keine Möglichkeit, mit Hilfe eines Messgeräts, einer Messanzeige und dergleichen festzustellen, wie stark die Reaktion tatsächlich ist. Vielmehr misst der Computer 1 jeweils, wie stark die tatsächliche Reaktion von dem Sollwert abweicht, wie stark beispielsweise die tatsächliche Druckkraft von der Soll-Druckkraft abweicht. Reagiert die Testperson beispielsweise positiv auf einen Sinnesreiz, so fällt die Reaktion üblicherweise stärker aus als dann, wenn die Testperson negativ auf einen Sinnesreiz reagiert. Mit der beschriebenen Sequenz kann in automatisierter Weise ein Datensatz gewonnen werden, der Aufschluss darüber gibt, wie die Testperson auf gewisse Sinnesreize reagiert, und der der Aufschluss über den psychosomatischen Zustand der Testperson geben kann.

Zum Festlegen der Sequenz von Sinnesreizen ist der einleitende Verfahrensschritt S9 bestimmt. Die Sequenz kann fest vorgegeben sein, so dass der Benutzer die Sequenz durch Drücken eines Startknopfes auslösen kann. Der Benutzer kann auch zwischen einer Anzahl von unterschiedlichen Sequenzen aussuchen. Hierzu kann der Benutzer mit Hilfe der Tastatur 4 und einer nicht dargestellten Maus in einer Datenbank eine Sequenz auswählen, wozu eine Menüsteuerung auf dem Bildschirm 3 vorgesehen sein kann. Der Benutzer kann die Sequenz auch individuell, beispielsweise in Anpassung an die Testperson, auswählen. Hierzu können dem Benutzer auf dem Bildschirm 3 Symbole für jeweilige Testreize angezeigt werden, beispielsweise eine Textbeschreibung, ein Bild oder dergleichen. Mit Hilfe der Tastatur 4 und einer nicht dargestellten Maus kann der Benutzer diese Symbole auswählen und individuell zu einer Sequenz zusammenfügen. Die ausgewählte Sequenz wird anschließend im Arbeitsspeicher des Computers 1 oder auf einem Datenträger abgespeichert.

Beim Festlegen der Sequenz im Verfahrensschritt S9 kann der Benutzer auch vorgeben, ob die Sequenz mit fester Geschwindigkeit oder mit variabler Geschwindigkeit ablaufen soll. Der Benutzer kann auch einen Schwellenwert vorgeben, den die Stärke des erfassten elektrischen Signals jeweils überschreiten muss, bevor ein nächster Sinnesreiz aus der Sequenz von Sinnesreizen dargeboten wird.

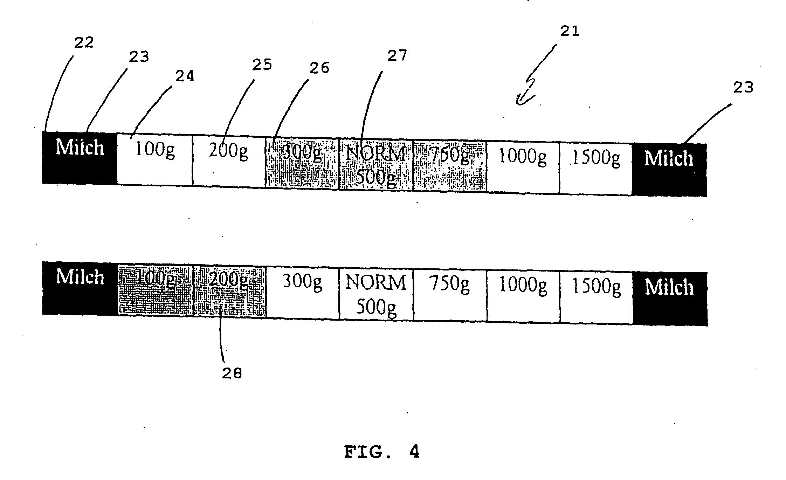

Bevor die Sequenz der Testperson dargeboten wird, sollte der Testperson ein Sollwert für die Stärke der körperlichen oder physiologischen Aktivität vorgegeben oder antrainiert werden. Hierzu wird in dem einleitenden Verfahrensschritt S10 auf dem Bildschirm 3 des Computers 1 visuell die Stärke der aktuellen körperlichen oder physiologischen Aktivität dargestellt. Soll die Testperson beispielsweise auf die in den Figuren 1 dargestellten Drucksensoren drücken, so wird auf dem Bildschirm 3 des Computers 1 die ausgeübte Druckkraft dargestellt, beispielsweise in Form eines Zeigers oder eines Punktes, der auf einer Skala die aktuelle Druckkraft anzeigt. Außerdem wird in dem Verfahrensschritt S10 in vergleichbarer Weise auch ein Sollwert angezeigt, den die Stärke der zu messenden körperlichen oder physiologischen Aktivität der Testperson in Reaktion auf einen Sinnesreiz erreichen soll. Soll die Testperson beispielsweise in Reaktion auf einen Sinnesreiz mit einer Druckkraft von etwa 500 g auf einen Drucksensor drücken, so kann auf dem Bildschirm 3 eine Skala dargestellt werden, wie sie in Figur 4 gezeigt ist, mit einer Einfärbung im Bereich des Sollwerts (500 g).

In dem Verfahrensschritt S10 übt die Testperson nun die zu messende körperliche oder physiologische Aktivität eine Anzahl von Malen aus. Beispielsweise soll die Testperson eine Anzahl von Malen mit einer Druckkraft von 500 g (= Soll-Druckkraft) auf den Drucksensor drücken und sich an das Gefühl gewöhnen, mit dieser Soll-Druckkraft zu drücken.

Zum Gewöhnen an diesen Sollwert kann auch die Anzeige 17 der in Figur 1 gezeigten Briefwaage 14 unmittelbar dienen. In diesem Fall muss sich die Testperson während des Ablaufens der Sequenz dazu zwingen, nicht auf die Skala 17 zu blicken. Die Skala 17 der Briefwaage kann auch während des Ausführens der Sequenz automatisch verdeckt werden.

Nach Durchlaufen der Verfahrensschritte S9 bis S11 geht das Verfahren über zu Schritt S1 gemäß Fig. 2.

Der Verfahrensschritt S10 zum Trainieren der Testperson auf einen vorgegebenen Sollwert kann auch während die Sequenz dargeboten wird, ausgeführt werden (Verfahrensschritt S8).

Es ist bekannt, dass Patienten im Reverse sein können. Solche Personen reagieren auf Reize atypisch. Reagieren Testpersonen beispielsweise üblicherweise auf positive Reize stärker und auf negative Reize schwächer, so ist dies bei den Testpersonen im Reverse genau anders herum, d.h. diese reagieren auf positive Reize schwächer und auf negative Reize stärker. Für Personen im Reverse würde das vorstehend beschriebene Testverfahren falsche Ergebnisse liefern.

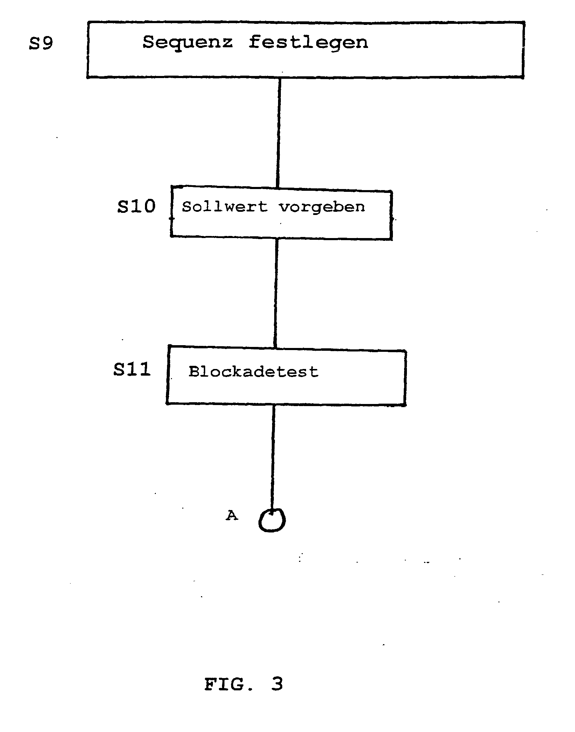

Um Testpersonen im Reverse herauszufiltern, kann vor dem Ausführen der Verfahrensschritte S1 bis S7 gemäß Figur 2 und im Anschluss an die Schritte S9 und S10 der Verfahrensschritt S11 gemäß Figur 3 ausgeführt werden.

Wurde die Testperson also im Schritt S10 daran gewöhnt, die zu messende körperliche oder physiologische Aktivität mit einer vorgegebenen Stärke auszuführen, beispielsweise mit einer vorgegebenen Druckkraft auf einen Drucksensor zu drücken, so werden der Testperson im Verfahrensschritt S11 anschließend gezielt objektiv falsche Informationen dargeboten und es wird nun ermittelt, ob die Testperson auf eine solche objektiv falsche Information stärker oder schwächer reagiert. Reagiert die Testperson auf eine falsche Information stärker, so handelt es sich um eine Testperson im Reverse. Um das Reverse-Verhalten objektiver beurteilen zu können, kann dieser Test mehrmals durchgeführt werden.

Um objektiv falsche Informationen zu generieren, kann der Computer 1 mit einer Datenbank mit für die Testperson spezifischen Datensätzen verknüpft sein. Diese kann objektiv richtige Information enthalten, beispielsweise belegbare Angaben zur Person, wie beispielsweise Name, Vorname, Geburtsdatum, wichtige Lebensdaten, Krankheiten etc. Der Computer 1 generiert nun auf der Grundlage dieser objektiv richtigen Informationen objektiv falsche Informationen. Hierzu kann vorgesehen sein, dass der Computer Sätze generiert und auf dem Bildschirm 3 anzeigt. Zum Bilden dieser Sätze wird die in der für die Testperson spezifischen Datenbank enthaltene objektiv richtige Information so abgeändert, dass die Information objektiv falsch ist. Beispielsweise kann der Vorname der für die Testperson in einen anderen Vornamen geändert werden, der also objektiv falsch ist. Anschließend wird der Testperson ein mit diesem falschen Vornamen gebildeter Satz dargestellt und es wird die Reaktion der Testperson auf diesen objektiv falschen Satz ermittelt.

Zum Generieren dieser objektiv falschen Information können sämtliche Angaben zur Testperson herangezogen werden. Statt dass die für die Testperson spezifische Datenbank objektiv richtige Informationen enthält, kann diese auch bereits objektiv falsche Informationen enthalten, die entweder von der Testperson selbst oder von einer dritten Person in Kenntnis der objektiv richtigen Information, betreffend die Testperson, eingegeben werden können. Enthält die für die Testperson spezifische Datenbank bereits objektiv falsche Information, braucht der Computer 1 zum Erzeugen von objektiv falscher Information die aus dieser Datenbank bezogene Informationen nicht verfälschen, kann diese vielmehr direkt übernehmen.

Bei einer weiteren Ausführungsform kann vorgesehen sein, dass auch der Reversetest gemäß Verfahrensschritt S11 stets dann im Anschluss an eine neue Vorgabe eines Sollwerts (Verfahrensschritt S8) in der Schleife S1 bis S5 ausgeführt wird, wenn also im Verfahrensschritt S4, anhand der ermittelten körperlichen oder physiologischen Aktivität der Testperson in Reaktion auf einen dargebotenen Sinnesreiz festgestellt wird, dass ein neuer Sollwert vorgegeben werden soll.

Der vorgenannte Reversetest kann auch dazu verwendet werden, um sogenannte blockierte Testpersonen herauszufiltern, die blockiert sind, also keine bevorzugt positive oder negative Reaktion zeigen. Hierzu wird der Testperson eine objektiv falsche Information, wie zuvor beschrieben, dargeboten. Wird festgestellt, dass die Testperson nicht stärker oder schwächer reagiert, sondern stets gleich, so kann die Vorrichtung gemäß der Erfindung feststellen, dass die Testperson blockier ist. In einem solchen Fall kein ein Warnhinweis auf dem Bildschirm 3 des Computers 1 angezeigt werden und/oder das Verfahren gemäß der Erfindung abgebrochen werden.

Figur 4 zeigt ein Beispiel für eine grafische Auswertung der Reaktion einer Testperson auf den Sinnesreiz "Milch", der beispielsweise als gesprochenes Wort, als Bild oder als Text auf dem Bildschirm 3 des Computers 1 dargeboten werden kann. Der Figur 4 liegt zugrunde, dass die Testperson mit einer Soll-Druckkraft von 500 g auf einen Drucksensor drükken soll.

Gemäß Figur 4 umfasst die Anzeige 21 ein Feld 22, auf dem der dargebotene Sinnesreiz 23 schematisch dargestellt wird, beispielsweise als Wort oder als Bild. Außerdem umfasst die Anzeige 21 eine Anzahl Felder 24 mit einer Anzeige 25 für die Stärke der Druckkraft, die dem jeweiligen Feld 24 entspricht, wobei die Werte der Anzeigen 25 in der Figur 4 von links nach rechts zunehmen von 100 g bis 1500 g.

Dem oberen Teil von Figur 4 liegt zugrunde, dass die Testperson auf den Reiz "Milch" einige Male mit einer Druckkraft im Bereich von 300 g bis 750 g reagiert hat, also in etwa gleich verteilt stärker und schwächer. Die Anzeige 21 wandelt diese Information in eine neutrale Anzeige um, worin der Bereich um die Soll-Druckkraft von 500 g farbig oder schraffiert hinterlegt ist und zwar symmetrisch um die Soll-Druckkraft, so dass mit einem Blick auf die Anzeige 21 erkannt werden kann, dass keine besondere Referenz in der Reaktion der Testperson vorliegt.

Dem unteren Teil von Figur 4 liegt zugrunde, dass die Testperson eine Anzahl von Malen mit einer Druckkraft von 100 g bis 200 g auf den Reiz "Milch" reagiert hat, also zu schwach. In diesem Fall sind die Felder 24 mit den Stärken 100 g und 200 g farbig hinterlegt (28). Bevorzugt ist die Farbe in diesem Fall anderes gewählt als im oberen Teil von Figur 4, beispielsweise im unteren Teil rot und im oberen Teil grün. Mit einem Blick auf die Anzeige 21 kann man deshalb erkennen, dass die Testperson im oberen Fall neutral reagiert hat und im unteren Fall mit einer Präferenz hin zu schwächeren Druckkräften, was auf eine pathologische Abweichung hindeuten kann, beispielsweise dass sich die Person bei dem Reiz "Milch" unsicher fühlt oder gar allergisch reagiert.

Zusätzlich zu der farbigen Hinterlegung kann auch vorgesehen sein, dass jedes Mal dann, wenn die Testperson mit einer Stärke, die der Stärke eines Felds 24 entspricht, reagiert hat, in dem jeweiligen Feld 24 ein schwarzer Punkt oder ein ähnliches Symbol angezeigt wird, so dass man an der Häufung der Punkte zusätzliche Informationen über die Präferenzen der Testperson gewinnen kann.

Eine besonders bevorzugte Verwendung der Vorrichtung und des Verfahrens gemäß der Erfindung betrifft einen Allergietest, bei dem der Testperson eine Sequenz von visuellen und/oder akustischen Reizen und/oder Texten dargeboten wird, und die Reaktion der Testperson jeweils in Reaktion auf den Sinnesreiz ermittelt wird, beispielsweise wenn die Testperson auf die Briefwaage 14 drückt. Aus der Abweichung der ausgeübten Druckkraft von der Soll-Druckkraft 27 kann für die jeweiligen "dargebotenen" Testsubstanzen in einfacher Weise ermittelt werden, ob die Testperson unsicher reagiert oder nicht. Dies kann Rückschlüsse auf das Vorliegen einer möglichen Allergie auf die jeweilige Testsubstanz zulassen, ohne dass die Testperson der potentiell eine Allergie auslösenden Substanz tatsächlich physisch ausgesetzt werden müsste.

In besonders vorteilhafter Weise kann die vorliegende Erfindung realisiert werden durch die Kombination eines handelsüblichen Computers, insbesondere Laptops, mit einer Briefwaage, die über eine Schnittstelle, beispielsweise eine RS 232-Schnittstelle, verfügt, zum Auslesen der Messwerte der Briefwaage durch den Computer. Bei dieser Kombination kann auf handelsübliche Komponenten und Softwareprodukte zurückgegriffen werden.

Das Computerprogrammprodukt gemäß der Erfindung umfasst Programmkode- bzw. Softwarecodeabschnitte, die jeweils einen der vorstehend beschriebenen Verfahrensschritte ausführen, so dass das Verfahren gemäß der Erfindung ausgeführt werden kann, wenn das Computerprogrammprodukt bzw. die Software in den Arbeitsspeicher eines handelsüblichen Computers, vorzugsweise eines Laptops, oder allgemeiner eines Datenverarbeitungsmittels geladen wird.

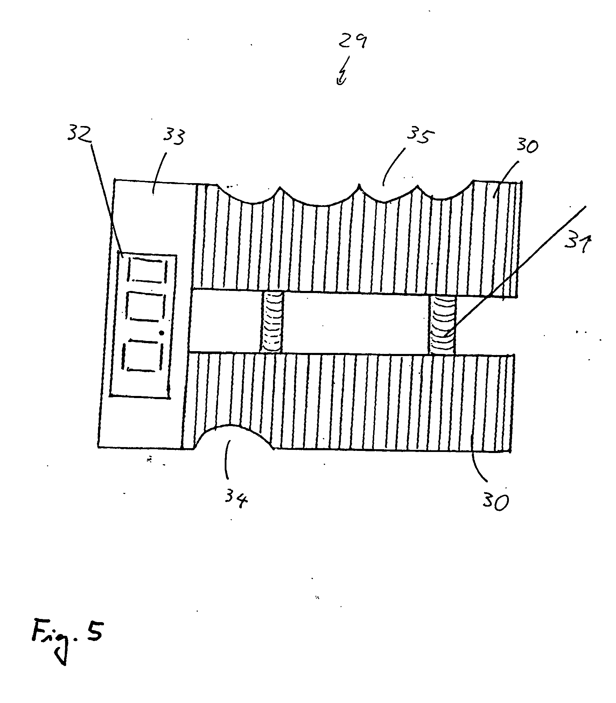

Die Figur 5 zeigt ein weiteres Beispiel einer bevorzugten Verwendungsform der erfindungsgemäßen Vorrichtung. Bei dieser ist das Datenverarbeitungsmittel vorzugsweise in die Vorrichtung 29 integriert und deshalb nicht dargestellt. Grundsätzlich entspricht die in Figur 5 gezeigte Vorrichtung 29 einem Handmuskeltestgerät in der Art üblicher Handmuskeltrainingsgeräte. Gemäß Figur 5 wird mit der Vorrichtung 29 gemessen, mit welcher Kraft die beiden Backen 30 der Vorrichtung 29 zusammengedrückt werden. Die Vorrichtung 29 umfasst hierzu zwei sich gegenüberliegenden Backen 30 mit Vertiefungen 35 für die Zeige- bis Ringfinger und einer Vertiefung 34 für den Daumen. Die beiden Backen 30 sind über die Abschnitte 31 miteinander verbunden. Die Abschnitte 31 können ein Rückstellmittel, beispielsweise eine Feder, umfassen, die die Backen 30 in die in Figur 5 dargestellte Ruhelage rückstellen. Die Abschnitte 31 können auch starre Abschnitte sein, wobei auf zumindest einer Stirnseite ein Druckaufnehmer (nicht dargestellt) vorgesehen ist, der die Stärke der Kraft misst, mit der die Testperson die Backe 30 zusammendrückt. An der Stirnseite befindet sich ein Abschnitt 33, der mit nur einer Backe verbunden ist. Die andere Backe kann beabstandet zu diesem oder in diesem gleitbeweglich angeordnet sein. In dem Abschnitt 33 befindet sich eine Anzeige 32, beispielsweise eine LCD-Anzeige. In den Abschnitt 33 integriert ist das nicht dargestellte Datenverarbeitungsmittel. Das Datenverarbeitungsmittel empfängt die von den nicht dargestellten Druck- bzw. Kraftaufnehmern gemessenen Signale, die die Stärke representieren, mit der die beiden Backen 30 von einer Testperson zusammengedrückt werden. Das Datenverarbeitungsmittel ist zur Ausführung der vorstehend genannten Verfahrensschritte ausgelegt.

Eine besonders bevorzugte Verwendungsform ist wie folgt: die Vorrichtung 29 eignet sich als mobile Testvorrichtung, beispielsweise zum Testen, ob von der Testperson ein Produkt gekauft werden soll oder nicht oder zum Treffen sonstiger Entscheidungen. Bevor die Testperson die Entscheidung zu treffen hat, drückt diese die Klemmbacken einige Male zusammen, beispielsweise zwei bis maximal zehn mal mit dem Ziel, die beiden Klemmbacken mit einer konstanten Kraft zusammenzudrücken. Auf diese Weise wird der Vorrichtung 29 ein Sollwert vorgegeben.

Anschließend drückt die Testperson jedes Mal dann, wenn eine Entscheidung zu treffen ist, die beiden Klemmbacken gegeneinander, wobei die nicht dargestellten Druck- bzw. Kraftaufnehmer, die dabei auftretenden Kräfte messen und anders nicht dargestellte Datenverarbeitungsmittel weiterleiten. Durch Vergleichen mit dem zuvor ermittelten Sollwert kann das Datenverarbeitungsmittel dann feststellen, ob die Stärke der Kraft bzw. des Druckes größer oder kleiner als zur zuvor ermittelte Sollwert ist. Das Ergebnis wird auf der Anzeige 32 angezeigt. Alternativ kann auf der Anzeige 32 auch lediglich die gemessene Stärke angezeigt werden. Daraus kann die Testperson ablesen, ob die Entscheidung tatsächlich getroffen werden soll oder nicht. Somit kommt die Vorrichtung 29 ohne Reizgenerator aus. Vielmehr werden die Sinnesreize von der Umgebung dargeboten.

Eine ganz bevorzugte Verwendungsform der Vorrichtung gemäß Figur 5 ist ein Einkaufstestgerät, das die Testperson beim Treffen von Kaufentscheidungen unterstützen soll. Somit betrifft die vorliegende Erfindung auch eine Vorrichtung zum Erfassen von Daten, betreffend den psychosomatischen Zustand einer Testperson, umfassend:
- einen Reizgenerator, um der Testperson Sinnesreize darzubieten,
- eine Steuereinrichtung, die den Reizgenerator so steuert, daß der Testperson automatisch eine Sequenz unterschiedlicher Sinnesreize dargeboten wird,
- einen Sensor, um von einer körperlichen oder physiologischen Aktivität der Testperson in Reaktion auf einen dargebotenen Sinnesreiz ein elektrisches Signal abzuleiten, und
- einen Komparator, um das elektrische Signal mit einem vorgebbaren Sollwert zu vergleichen.

Außerdem betrifft die vorliegende Erfindung ein Verfahren zum Erfassen von Daten betreffend den psychosomatischen Zustand einer Testperson, bei dem
- der Testperson Sinnesreize dargeboten werden und
- elektrische Signale von einer körperlichen oder physiologischen Aktivität der Testperson in Reaktion auf einen dargebotenen Sinnesreiz abgeleitet werden und die elektrischen Signale mit einem vorgebbaren Sollwert verglichen werden, wobei
- die Sinnesreize der Testperson in Form einer Sequenz unterschiedlicher Sinnesreize automatisch dargeboten werden.
- die Sinnesreize der Testperson in Form einer Sequenz unterschiedlicher Sinnesreize automatisch dargeboten werden.

Ferner betrifft die vorliegende Erfindung eine Vorrichtung zum Messen einer körperlichen oder physiologischen Aktivität einer Testperson und zum Beurteilen des psychosomatischen Zustands der Testperson, umfassend:
- einen Reizgenerator, um der Testperson Sinnesreize darzubieten,
- eine Steuereinrichtung, die den Reizgenerator so steuert, dass der Testperson automatisch eine Sequenz unterschiedlicher Sinnesreize dargeboten wird,
- einen Sensor, um von einer körperlichen oder physiologischen Aktivität der Testperson in Reaktion auf einen dargebotenen Sinnesreiz ein elektrisches Signal abzuleiten, gekennzeichnet durch
- eine Sollwerteinheit, die eine visuelle Anzeige zum Anzeigen der Stärke der körperlichen oder physiologischen Aktivität der Testperson in Reaktion auf einen Sinnesreiz und eine visuelle Anzeige zum Vorgeben eines Sollwerts aufweist, den die Stärke einer körperlichen oder physiologischen Aktivität der Testperson in Reaktion auf einen Sinnesreiz erreichen soll und
- einen Komparator, um das elektrische Signal mit einem vorgebbaren Sollwert zu vergleichen.

Nachfolgend werden weitere bevorzugte Verfahren beispielhaft angeführt:
A) Verfahren zum Messen einer körperlichen oder physiologischen Aktivität einer Testperson und zum Beurteilen des psychosomatischen Zustands der Testperson, bei dem
   - der Testperson Sinnesreize dargeboten werden und
   - elektrische Signale von einer körperlichen oder physiologischen Aktivität der Testperson in Reaktion auf einen dargebotenen Sinnesreiz abgeleitet werden und die elektrischen Signale mit einem vorgebbaren Sollwert verglichen werden, wobei
   - die Sinnesreize der Testperson in Form einer Sequenz unterschiedlicher Sinnesreize automatisch dargeboten werden, und
   - ein Sollwerteinstellschritt ausgeführt wird, bei dem der Testperson die aktuelle Stärke der körperlichen oder physiologischen Aktivität und ein Sollwert für die Stärke der körperlichen oder physiologischen Aktivität visuell angezeigt wird.
B) Verfahren nach Abschnitt A, bei dem die Sequenz akustische und/oder visuelle Sinnesreize und/oder Texte umfasst, die fest vorgegeben sind oder individuell in eine Datenbank für die Testperson eingebbar sind.
C) Verfahren nach Abschnitt B oder C, bei dem die Sequenz von Sinnesreizen mit konstanter Geschwindigkeit oder mit vorgebbar variabler Geschwindigkeit abläuft.
D) Verfahren nach einem der Abschnitte A bis C, bei dem ein nächster Reiz aus der Sequenz von Sinnesreizen erst dann dargeboten wird, wenn die Stärke eines in Reaktion auf einen vorhergehenden Reiz erfassten elektrischen Signals einen vorgegebenen Schwellenwert übersteigt.
E) Verfahren nach einem der Abschnitte A bis D, bei dem zum Erfassen der körperlichen Aktivität eine Druckkraft erfasst wird, die die Testperson auf einen Drucksensor (12, 15), insbesondere eine Briefwaage (14), ausübt.
F) Verfahren nach einem der Abschnitte A bis E, bei dem elektrische Signale, die während der Darbietung eines Sinnesreizes und/oder in einem vorbestimmten Zeitintervall nach der Darbietung eines Sinnesreizes erfasst werden, digitalisiert und zwischengespeichert werden.
G) Verfahren nach einem der Abschnitte A bis G, bei dem die digitalen Signale graphisch aufbereitet werden und/oder als Datensatz, der für die Testperson spezifisch ist, gespeichert werden.
H) Verfahren nach einem der Abschnitte A bis G, bei dem der Sollwerteinstellschritt jedes Mal dann ausgeführt wird, wenn die Abweichung der Stärke der körperlichen oder physiologischen Aktivität von dem Sollwert einen vorgegebenen Schwellenwert eine Anzahl von Malen überschreitet.
I) Verfahren nach einem der Abschnitte A bis H, bei dem der Testperson zunächst eine objektiv falsche Information dargeboten wird und die Stärke der körperlichen oder physiologischen Aktivität in Reaktion auf die falsche Information erfasst wird.
J) Verfahren nach dem Abschnitt I, bei dem die objektiv falsche Information aus einer Datenbank testpersonenspezifischer Datensätze abgeleitet wird.

## Patentansprüche

1. Vorrichtung zum Messen einer körperlichen oder physiologischen Aktivität einer Testperson und zum Beurteilen des psychosomatischen Zustands der Testperson, umfassend:
- einen Reizgenerator (2, 3), um der Testperson Sinnesreize darzubieten,
- eine Steuereinrichtung (1), die den Reizgenerator (2, 3) so steuert, dass der Testperson automatisch eine Sequenz unterschiedlicher Sinnesreize dargeboten wird,
- einen Sensor (12, 15, 18-20), um von einer körperlichen oder physiologischen Aktivität der Testperson in Reaktion auf einen dargebotenen Sinnesreiz ein elektrisches Signal abzuleiten, und
einen Komparator (1), um das elektrische Signal mit einem vorgebbaren Sollwert zu vergleichen, wobei die Steuereinrichtung (1) eine Datenbank umfasst, die Datensätze für eine Sequenz fest vorgegebener akustischer und/oder visueller Reize und/oder Texte enthält, die der Testperson dargeboten werden,
**dadurch gekennzeichnet, dass**
die Steuereinrichtung (1) konfiguriert ist, um die Sequenz mit Hilfe eines Benutzers individuell aus den Datensätzen zusammenfügen zu können.

2. Vorrichtung nach Anspruch 1, bei der die Steuereinrichtung (1) den Reizgenerator (2, 3) dazu veranlasst, die Sequenz mit fest vorgegebener oder mit variabel vorgebbarer Geschwindigkeit ablaufen zu lassen.

3. Vorrichtung nach einem der vorhergehenden Ansprüche, bei der die Steuereinrichtung (1) den Reizgenerator (2, 3) so steuert, dass ein nächster Reiz aus der Sequenz von Sinnesreizen erst dann dargeboten wird, wenn die Stärke eines in Reaktion auf einen vorhergehenden Reiz erfassten elektrischen Signals einen vorgegebenen Schwellenwert überschreitet.

4. Vorrichtung nach einem der vorhergehenden Ansprüche, bei der der Sensor (12, 15, 18-20) ein Drucksensor (12, 15), insbesondere eine Briefwaage (14), ist, wobei ein A/D-Wandler (6) die erfassten elektrischen Signale in digitale Signale wandelt.

5. Vorrichtung nach dem vorhergehenden Anspruch, wobei eine Prozessoreinheit (1) vorgesehen ist, um die digitalen Signale graphisch aufzubereiten und/oder als Datensatz zu speichern.

6. Vorrichtung nach einem der vorhergehenden Ansprüche, weiterhin umfassend eine Sollwerteinheit, die eine visuelle Anzeige zum Anzeigen der Stärke der Körperlichen oder Physiologischen Aktivität der Testperson in Reaktion auf einen Sinnesreiz und eine Visuelle Anzeige zum Vorgeben eines Sollwertes aufweist, den die Stärke einer körperlichen oder physiologischen Aktivität der Testperson in Reaktion auf einen Sinnesreiz erreichen soll.

7. Vorrichtung nach dem vorhergehenden Anspruch, bei der die Steuereinrichtung (1) die Sollwerteinheit jedes Mal ansteuert, wenn die Abweichung der Stärke der körperlichen Aktivität von dem Sollwert einen vorgegebenen Schwellenwert eine Anzahl von Malen überschreitet.

8. Vorrichtung nach Anspruch 6 oder 7, bei der die Steuereinrichtung (1) ausgelegt ist, um der Testperson eine objektiv falsche Information darzubieten und um die Stärke der körperlichen oder physiologischen Aktivität in Reaktion auf die falsche Information mit dein Schwellenwert zu vergleichen.

9. Vorrichtung nach dem vorhergehenden Anspruch, bei der die Steuereinrichtung (1) mit der Datenbank mit testpersonenspezifischen Datensätzen verknüpft ist, so dass die objektive falsche Information aus der Datenbank mit testpersonenspezifischen Datensätzen abgeleitet wird.

10. Verfahren zum Messen einer körperlichen oder physiologischen Aktivität einer Testperson und zum Beurteilen des psychosomatischen Zustands der Testperson, bei dem
- der Testperson Sinnesreize (2) dargeboten werden und
- elektrische Signale von einer körperlichen oder physiologischen Aktivität der Testperson in Reaktion auf einen dargebotenen Sinnesreiz abgeleitet werden und die elektrischen Signale mit einem vorgebbaren Sollwert verglichen werden, wobei
- die Sinnesreize der Testperson in Form einer Sequenz unterschiedlicher Sinnesreize automatisch dargeboten werden, wobei die Sequenz akustische und/oder visuelle Sinnesreize und/oder Texte umfasst, die fest vorgegeben sind oder individuell in eine Datenbank für die Testperson eingebbar sind,
**dadurch gekennzeichnet, dass**
die Sequenz mit Hilfe eines Benutzers individuell aus den akustischen und/oder visuellen Sinnesreizen und/oder Texten zusammengefügt wird.

11. Verfahren nach Anspruch 10, bei dem die Sequenz von Sinnesreizen mit konstanter Geschwindigkeit oder mit vorgebbar variabler Geschwindigkeit abläuft.

12. Computerprogrammprodukt, umfassend Programmkodeabschnitte, die direkt in den Arbeitsspeicher eines mit zumindest einem Sensor verbundenen Datenverarbeitungsmittels ladbar sind, so dass das Datenverarbeitungsmittel die Verfahrensschritte nach Anspruch 10 oder 11 ausführt.

13. Computerprogrammprodukt mit Programmkodeabschnitten nach dem vorhergehenden Anspruch, die auf einem computerlesbaren Datenträger gespeichert sind.

## Claims

1. Device for measuring a physical or physiological activity of a subject and for assessing the psychosomatic state of the subject, comprising:
- a stimulus generator (2, 3) for presenting the subject with sensory stimuli,
- a control device (1) which controls the stimulus generator (2, 3) in such a way that the subject is automatically presented with a sequence of different sensory stimuli,
- a sensor (12, 15, 18-20) for deriving an electrical signal from a physical or physiological activity of the subject in response to a presented sensory stimulus, and
- a comparator (1) for comparing the electrical signal with a predefinable reference value, whereby the control device (1) comprises a databank containing data sets for a sequence of fixedly predefined acoustic and / or visual stimuli and / or texts which are presented to the subject,
**characterised in that**
the control device (1) is configured to be able to put together the sequence with the aid of a user individually from the data sets.

2. Device according to claim 1, wherein the control device (1) triggers the stimulus generator (2, 3) to allow execution of the sequence at fixedly predefined or at variably predefinable speed.

3. Device according to one of the preceding claims, wherein the control device (1) controls the stimulus generator (2, 3) in such a way that a next stimulus from the sequence of sensory stimuli is only presented when the strength of an electrical signal detected in response to a preceding stimulus exceeds a predefined threshold value.

4. Device according to one of the preceding claims, wherein the sensor (12, 15, 18-20) is a pressure sensor (12, 15), in particular a letter scale (14), wherein an A/D converter (6) converts the detected electrical signals into digital signals.

5. Device according to the preceding claim, wherein a processor unit (1) is provided for preparing the digital signals graphically and / or storing them as a data set.

6. Device according to one of the preceding claims, further comprising a reference value unit which comprises a visual display for displaying the strength of the physical or physiological activity of the subject in response to a sensory stimulus and a visual display for predefining a reference value which should be reached by the strength of a physical or physiological activity of the subject in response to a sensory stimulus.

7. Device according to the preceding claim, wherein the control device (1) controls the reference value unit each time the deviation in the strength of the physical activity from the reference value exceeds a predefined threshold value a number of times.

8. Device according to claim 6 or 7, wherein the control device (1) is designed to present objectively false information to the subject and to compare the strength of the physical or physiological activity in response to the false information with the threshold value.

9. Device according to the preceding claim, wherein the control device (1) is connected to the database with subject-specific data sets, so that the objectively false information is derived from the database with subject-specific data sets.

10. Method for measuring a physical or physiological activity of a subject and for assessing the psychosomatic state of the subject, in which
- sensory stimuli (2) are presented to the subject and
- electrical signals are derived from a physical or physiological activity of the subject in response to a presented sensory stimulus and the electrical signals are compared with a predefinable reference value, wherein
- the sensory stimuli are automatically presented to the subject in the form of a sequence of different sensory stimuli, wherein the sequence comprises acoustic and / or visual sensory stimuli and / or texts which are fixedly predefined or can be input individually into a database for the subject,
**characterised in that**
the sequence is put together with the aid of a user individually from the acoustic and / or visual sensory stimuli and / or texts.

11. Method according to claim 10, wherein the sequence of sensory stimuli is executed at constant speed or at predefinably variable speed.

12. Computer program product, comprising program code segments which can be loaded directly into the working memory of data processing means connected to at least one sensor in such a way that the data processing means execute the method steps according to claim 10 or 11.

13. Computer program product with program code segments according to the preceding claim which are stored on a computer-readable data carrier.

## Revendications

1. Dispositif pour mesurer une activité physique ou physiologique d'une personne et pour évaluer l'état psychosomatique de cette personne, comprenant:
- un générateur de stimulations (2, 3), pour présenter à la personne des stimulations sensorielles,
- un dispositif de commande (1), qui commande le générateur de stimulations (2, 3) de façon à présenter automatiquement à la personne une séquence de stimulations sensorielles différentes,
- un capteur (12, 15, 18-20), pour déduire un signal électrique à partir d'une activité physique ou physiologique de la personne en réaction à une stimulation sensorielle présentée, et
- un comparateur (1), permettant de comparer le signal électrique à une valeur de consigne prédéterminable, le dispositif de commande (1) comportant une banque de données, qui contient des ensembles de données pour une séquence de stimulations acoustiques et/ou visuelles et/ou de textes fixes prédéterminés, qui sont présentés à la personne,
**caractérisé en ce que**
- le dispositif de commande (1) est configuré de façon à pouvoir composer la séquence à partir des ensembles de données à l'aide d'un utilisateur individuellement.

2. Dispositif selon la revendication 1, dans lequel le dispositif de commande (1) active le générateur de stimulations (2, 3) de façon à exécuter la séquence avec une vitesse fixe prédéterminée ou avec une vitesse variable prédéterminable.

3. Dispositif selon l'une quelconque des revendications précédentes, dans lequel le dispositif de commande (1) commande le générateur de stimulations (2, 3) de façon à ne présenter une stimulation suivante de la séquence de stimulations sensorielles que lorsque l'intensité d'un signal électrique, détecté en réaction à une stimulation précédente, dépasse une valeur de seuil prédéterminée.

4. Dispositif selon l'une quelconque des revendications précédentes, dans lequel le capteur (12, 15, 18-20) est un capteur de pression (12, 15), en particulier un pèse-lettres (14), dans lequel un convertisseur A/D (6) convertit les signaux électriques détectés en signaux numériques.

5. Dispositif selon la revendication précédente, dans lequel il est prévu une unité de traitement (1) permettant de préparer graphiquement les signaux numériques et/ou de les mémoriser sous forme d'ensembles de données.

6. Dispositif selon l'une quelconque des revendications précédentes, comprenant en outre une unité de valeur de consigne, qui comporte un affichage visuel pour afficher l'intensité de l'activité physique ou physiologique de la personne en réaction à une stimulation sensorielle et un affichage visuel pour prédéterminer une valeur de consigne, que l'intensité d'une activité physique ou physiologique de la personne doit atteindre en réaction à une stimulation sensorielle.

7. Dispositif selon la revendication précédente, dans lequel le dispositif de commande (1) active l'unité de valeur de consigne chaque fois que l'écart entre l'intensité de l'activité physique et la valeur de consigne dépasse un certain nombre de fois une valeur de seuil prédéterminée.

8. Dispositif selon la revendication 6 ou 7, dans lequel le dispositif de commande (1) est conçu de façon à présenter à la personne une information objectivement fausse et à comparer l'intensité de l'activité physique ou physiologique en réaction à l'information fausse avec la valeur de seuil.

9. Dispositif selon la revendication précédente, dans lequel le dispositif de commande (1) est relié à la banque de données contenant des ensembles de données spécifiques à la personne, de telle manière que l'information objectivement fausse soit déduite de la banque de données contenant des ensembles de données spécifiques à la personne.

10. Procédé pour mesurer une activité physique ou physiologique d'une personne et pour évaluer l'état psychosomatique de la personne, dans lequel
- on présente à la personne des stimulations sensorielles (2), et
- on déduit des signaux électriques à partir d'une activité physique ou physiologique de la personne en réaction à une stimulation sensorielle présentée et on compare les signaux électriques à une valeur de consigne prédéterminable, dans lequel
- on présente automatiquement à la personne les stimulations sensorielles sous la forme d'une séquence de stimulations sensorielles différentes, la séquence comprenant des stimulations sensorielles acoustiques et/ou visuelles et/ou des textes, qui sont prédéterminés de façon fixe ou qui peuvent être introduits individuellement pour la personne dans une banque de données,
**caractérisé en ce que**
- on compose la séquence individuellement à l'aide d'un utilisateur, à partir des stimulations sensorielles acoustiques et/ou visuelles et/ou des textes.

11. Procédé selon la revendication 10, dans lequel on exécute la séquence de stimulations sensorielles avec une vitesse constante ou avec une vitesse variable prédéterminable.

12. Programme d'ordinateur, comprenant des sections de code de programme, qui peuvent être chargées directement dans la mémoire de travail d'un moyen de traitement de données relié à au moins un capteur, de telle manière que le moyen de traitement de données exécute les étapes du procédé selon la revendication 10 ou 11.

13. Programme d'ordinateur comprenant des sections de code de programme selon la revendication précédente, qui sont mémorisées sur un support de données lisible par un ordinateur.
